# EUROPEAN PATENT APPLICATION

(11) **EP 1 790 722 A1**
(43) Date of publication of application: **30.05.2007**
(21) Application number: 06256014.9
(22) Date of filing: 24.11.2006
(51) Int. Cl.: C12N 15/09

(54) **Recombinant bacteria and uses thereof**

(30) Priority: 24.11.2005 GB 0523949
(71) Applicant: El-Gewely, Mohammed Raafat, 9024 Tomasjord (NO)
(72) Inventor: El-Gewely, Mohammed Raafat, 9024 Tomasjord (NO)
(74) Representative: Gardner, Rebecca Katherine

(57) **Abstract**

The present inventions provides recombinant bacteria selected from K37, RR1, W3110, a mutant of any thereof, and a strain derived from one of these strains, wherein said bacterium contains a gene which encodes an exogenous polymerase and which is under the control of an inducible promoter, as well as uses of said bacteria and methods of protein production involving such bacteria.

## Description

The present invention relates to recombinant bacteria and their uses in the production of protein, in particular protein production through expression from exogenous nucleic acid. In a further aspect, the invention also relates to a method of producing a recombinant peptide or protein using a recombinant bacterium.

Proteins are important biomolecules with a large range of useful applications. Many proteins such as hormones have therapeutic applications. In Drug development active proteins are required as a target for drug development. Proteins may also be used in food and/or feed products. Pure protein is also required for many industrial and/or experimental uses. Depending on the specific application, the amount of protein required may be large. It may be desirable to have the protein present in its functional configuration and/or in as pure as possible form.

Many recombinant proteins are produced by expression of exogenous nucleic acid in a host bacterium. It is often desirable to express such proteins in large quantities. Furthermore, it is important that the expressed protein folds correctly, i.e. that it adopts the appropriate three dimensional configuration required for it to function properly.

One of the most commonly used *E. coli* strains for expression of recombinant protein is BL21 and derivatives thereof. This strain is widely accepted to be one of the most suitable strains for producing recombinant proteins, in particular because it is deficient in both *lon* and *ompT* proteases. There is therefore a prejudice in the art against the use of other strains for the production of recombinant protein, particularly strains not deficient in these proteases, as proteases may degrade (partially or fully) recombinant proteins and may hence negatively affect the yield of functional recombinant protein. Thus, although many other strains of *E.coli* are known and some of these have occasionally been used to express protein, only a few strains such as BL21 and its derivates were considered to be useful for the production of recombinant protein.

The present inventor has found that both the amount of recombinant protein produced and the ability to support correct folding varies significantly among bacterial strains, and he has been able to identify strains which allow the production of high levels of functional recombinant protein.

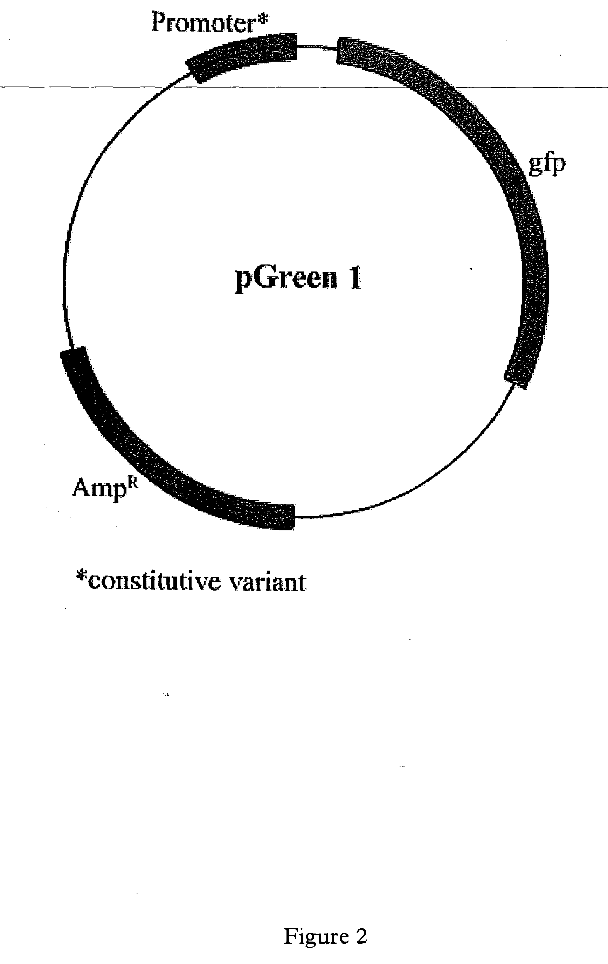

The expression of recombinant protein was assayed in a number of different strains of *Escherichia coli* by measuring the intensity of fluorescence emitted from cultures of different E. *coli* strains expressing green fluorescent protein from an exogenous gene on the pGreen1 construct (Figure 2).

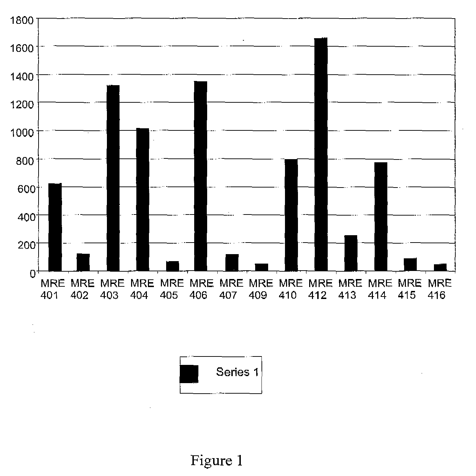

The results are summarised in Figure 1, which shows that there is very significant variation in the amount of fluorescence recorded from different *E.coli* strains expressing GFP. The higher the fluorescence intensity, the larger is the amount of correctly folded green fluorescent protein produced by the host bacterium.

The three E. coli strains which were most efficient at producing high amounts of active exogenous protein were termed MRE403, MRE406 and MRE412. These correspond to E.coli strains K37, W3110 and RR1. Some characteristics of these strains are listed in Table 1.

**Table 1**

| Strain | main genotype features | Reference |
|---|---|---|
| K37 | *F⁻ himA⁺* | Miller and Friedman 1980 Cell, vol 20, pp 711-719 |
| W3110 | K12 prototroph | Dr H. Aiba, Japan |
| RR1 | F⁻ *pro leu thi lacY* Str^{r} rₖ⁻mₖ⁻ | Bolivar et al. 1977, Gene 2, pp95-113 |

The present inventor realised the exceptional potential of strains K37 and RR1 for the production of recombinant protein and in particular for large scale/industrial protein production.He also realised that these strains would have even greater utility in the expression of recombinant polypeptide or protein if they contained nucleic acid encoding a specialised polymerase.

The utility of strain W3110 was also recognised.

Strains K37 RR1 and W3110 have not previously been considered to be useful in the production of recombinant protein, so there had previously been no motivation to modify these strains to allow controlled expression of exogenous nucleic acid.

Of particular interest was the gene encoding the T7 polymerase. This is carried by the DE3 lysogen, and insertion of this lysogen into these strains was carried out as described in Example 1.

T7 polymerase does not naturally occur in *E. coli.* It is a viral polymerase, naturally encoded by the bacteriophage T7, which recognises the T7 promoter, which is a very short recognition sequence. Any genes placed under the control of a T7 promoter can be transcribed by T7 polymerase. This system is highly specific, i.e. transcription from the T7 promoter only occurs via T7 polymerase.

Expression of T7 polymerase encoded by DE3 is under the control of the *lacUV5* promoter and is thus controlled by the lac repressor, i.e. expression of T7 polymerase may be regulated through use of a suitable inducer. The skilled person will be familiar with the lac regulation system. The most appropriate inducer for activating (de-repressing) transcription from the *lacUV5* promoter is IPTG. In the absence of any IPTG (or other inducer), expression of T7 polymerase will be minimal or completely repressed, and consequently there will be little or no expression of any exogenous nucleic acid placed under the control of the T7 promoter. Conversely, in the presence of a suitable inducer such as IPTG, expression of T7 polymerase will be high, allowing expression from the T7 promoter, i.e. expression of any genes placed under the control of a T7 promoter.

In the above discussion, expression of T7 polymerase is under the control of the lac repressor. However, the skilled person will be aware of other suitable control systems such as tetracycline-dependent promoters (examples include promoters induced by the presence of tetracycline and promoters repressed in the presence of tetracylcine), copper-inducible promoter, copper-repressive promoter, heat shock promoter, alcohol induced promoters e.g. methanol-inducibel promoters, steroid-regulated promoters and the like and these are also contemplated in the present invention.

The T7 system explained above is just an example of a suitable system. Although T7 polymerase is preferred, recombinant bacteria transformed instead or in addition to T7 with a different exogenous polymerase are also contemplated by the present invention. Preferably, the bacteria which are transformed to produce recombinant bacteria according to the invention are E. *coli* strains RR1, W3110 or K37 or mutants thereof, or a strain which has the identifying characteristics of one of these strains, or is a strain derived from one of these strains. More preferably, they are MRE403.DE3, MRE406.DE3 or MRE412.DE3 or mutants thereof, or a strain which has the identifying characteristics of one of these strains, or is a strain derived from one of these strains. Strains RR1 and K37 are especially preferred.

Suitable polymerase systems include for example Sp6 (native to Salmonella bacteriophage), and T3 (native to E. *coli* bacteriophage) polymerases. Any gene which it is desired to expressed may be placed under the control of the corresponding promoter, so e.g. when the Sp6 polymer is used, the gene of interest will be under the control of an Sp6 promoter.

By "exogenous nucleic acid" is meant nucleic acid which has been introduced into the bacterium through genetic manipulation. Typically, the exogenous nucleic acid will be located on a vector such as a plasmid vector. Thus, the term "exogenous nucleic acid" as used herein means a nucleic acid which has been introduced into the bacterium. Whether or not the bacterium naturally contains any nucleic acid which is similar or identical to the "exogenous nucleic acid" is immaterial. However, by "exogenous polymerase" is meant a polymerase which does not naturally occur in the host bacterium. It may be introduced into the bacterium through genetic manipulation. Thus, T7 polymerase is an "exogenous polymerase" because it does not naturally occur in E. coli.

By "recombinant polypeptide or protein" is meant any polypeptide or protein expressed from the exogenous nucleic acid. The term "target" polypeptide or protein is used interchangeably with the term "recombinant polypeptide or protein".

By "recombinant bacterium" is meant a bacterium which has been genetically modified through the introduction of foreign nucleic acid. By "foreign nucleic acid" is meant nucleic acid which does not naturally occur in that strain of bacterium. The foreign nucleic acid may be present in an extrachromosomal form such as on a vector, but preferably the foreign nucleic acid has been inserted into the bacterial chromosome.

The term "host bacterium" is used to denote a bacterium used for expressing exogenous nucleic acid.

"Identifying characteristics" include an ability to express an exogenous target protein placed under the control of a promoter which is recognised by a polymerase expressed by the bacterium but which is exogenous thereto; preferably expression of the target protein is at a level comparable with or superior to the level demonstrated for the three best strains in example 2 herein. Mutants and derived strains will likewise have this ability and will preferably be genotypically substantially identical (within coding and control sequences), e.g. at least 98%, 95% or 90% to the named strains."

Thus, in one aspect, there is provided a recombinant bacterium selected from K37, RR1, W3110, a mutant thereof and a strain derived from one of these strains, which has been modified to contain a gene which encodes an exogenous polymerase and which is under the control of an inducible promoter, preferably with the proviso that where the bacterium is a W3110 strain it has the identifying characteristics of the strain deposited under number NCTC13370.

Preferably, the exogenous polymerase is T7 polymerase. More preferably, the gene which encodes T7 polymerase and which is under the control of an inducible promoter is provided by the DE3 lysogen. Preferably, said gene is integrated into the bacterial chromosome. Most preferably, the recombinant bacterium has the identifying characteristics of a strain selected from MRE403.DE3, MRE406.DE3 and MRE412.DE3 which have been deposited with the National Collection of Type Cultures (NCTC) of 61 Colindale Avenue, London, NW9 5HT, United Kingdom on 11 October 2005 by Mr M Raafat El-Gewely under the Budapest Treaty, or a mutant thereof, or a strain derived there from. In particular, any genotypic variation from these deposited strains will not adversely affect the ability to these strains to express exogenous target proteins.

The deposited strains have been given the following Accession Numbers:

| ***Escherichia coli* Strain** | **Accession Number** |
|---|---|
| MRE403.DE3 | NCTC13369 |
| MRE406.DE3 | NCTC 13370 |
| MRE412.DE3 | NCTC13371 |

In a further aspect, there is provided the use of a recombinant bacterium as defined above in a method of recombinant protein production. Preferably, the bacterium has the identifying characteristics of a strain selected from MRE403.DE3, MRE406.DE3 and MRE412.DE3 or a mutant thereof, or a strain derived there from.

In a further aspect, there is provided a method of recombinant polypeptide or protein production. The method comprises the steps of
(a) introducing an expression vector into a recombinant bacterium selected from or derived from E. *coli* strains RR1, W3110 and K37, said bacterium containing a gene encoding an exogenous DNA polymerase under an inducible promoter, the expression vector comprising a polynucleotide sequence coding for a target peptide or protein under the control of a promoter which can be recognised by said exogenous DNA polymerase;
(b) culturing the recombinant bacterium under conditions in which expression of said exogenous DNA polymerase is turned on; and
(c) recovering the recombinant protein.

In another embodiment, there is provided a method of recombinant polypeptide or protein production. The method comprises the steps of:
(a) providing a recombinant bacterium selected form or derived from *E. coli* strains RR1, W3110 and K37 which contains a gene encoding an exogenous DNA polymerase under an inducible promoter and a polynucleotide sequence coding for a target polypeptide or protein under the control of a promoter which can be recognised by said exogenous DNA polymerase;
(b) culturing the recombinant bacterium under conditions in which expression of said exogenous DNA polymerase is turned on; and
(c) recovering the recombinant polypeptide or protein.

It will be understood that a promoter which "can be recognised" by a DNA polymerase is a promoter from which said DNA polymerase can initiate transcription. Preferably, said promoter is recognised by an exogenous DNA polymerase but is not recognised by any endogenous DNA polymerases. Most preferably, said promoter is specific for said exogenous polymerase and is not recognised by any other polymerases.

The skilled person will know how to "culture a bacterium under conditions in which expression of the DNA polymerase is turned on". As discussed above, inducible promoter systems are well known in the art and so for example when the inducible promoter is the *lacUV5* promoter, then "conditions in which expression of the DNA polymerase is turned on" will typically mean that a suitable amount of the inducer IPTG is added. When the inducible promoter is a heat-shock promoter, the conditions include exposing the bacteria to a heat shock, i.e. a temporary increase in temperature.

Way to recover the recombinant protein or polypeptide are known in the art and can be routinely performed by the skilled person. By way of example, the bacteria may be harvested, optionally washed, lysed and subjected to gel filtration, filtration, chromatography e.g. affinity chromatography or ion exchange chromatography and optionally subjected to dialysis. The protein may be concentrated by filtration or precipitation. All of these methods are standard procedures in the art.

Preferably, the DNA polymerase is T7 polymerase and the promoter is the T7 promoter. More preferably, the recombinant bacterium has the identifying characteristics of a strain selected from MRE403.DE3 and MRE412.DE3 or a mutant thereof, or a strain derived there from.

It is preferred that the exogenous polymerase is present within the chromosome of the host bacterium and the target protein is present on an expression vector which is introduced into said host bacterium.

Preferably, the expression vector contains
(a) a nucleotide sequence under the control of a promoter recognised by a specialised, i.e. exogenous to the host, DNA polymerase, preferably T7 polymerase; and preferably at least one of the features selected from:
b) tRNA genes ArgU, ArgW and IleX to improve heterologous expression of eukaryotic proteins in a prokaryotic host cell; and/or
c) an origin of replication which is compatible with most other origins of replication; and/or
d) a selectable marker.

Preferably, the selectable marker is an antibiotic resistance gene, e.g. a chloramphenicol or Kanamycin resistance gene.

More preferably, the vector for use in the above method has two or more of features (a) to (d), even more preferably it has all of these features.

The recombinant bacteria of the present invention are particularly suitable for use in a method of large-scale/industrial scale protein production. Thus, the method of protein production may be carried out in a total culture volume of more than 0.2, 0.4, 0.6, 0.8, 1, 2, 3, 4, 5, 6, 7, 8, 9 or 10 L, preferably more than 50 L, e.g. 60, 70, 80, 90 or 100 L, more preferably more than 100 L, e.g. more than 200, 300, 400, 500, 600, 700, 800 or 900 L, most preferably more than 1000 L, e.g. more than 1500, 2000, 2500 or 3000 L. Preferably, the method of protein production is carried out in a bioreactor, most preferably a tank bioreactor. The recombinant bacteria of the present invention are thus suitable for the production of large amounts of recombinant protein, e.g. the production of a total amount of at least 15 or 30 grams, preferably at least 40, 50, 60, 70, 80, 90 or 100 grams, more preferably at least 150, 200, 250, 300, 350, 400, 450, or 500 grams. The recombinant bacteria of the present invention are also particularly suitable for use in a method of soluble protein production and for use in the production of recombinant therapeutic proteins and/or active protein targets for drug discovery.

The method of protein production may be carried out as batch, fed-batch or continuous culture. In a preferred embodiment, a continuously stirred tank reactor (CSTR) is used. The recombinant bacteria may be present in suspension or they may be immobilised. In one embodiment, the method of protein production is carried out using a large scale immobilised cell bioreactor of the packed bed, fibrous bed or membrane type.

The bioreactor may monitor, control and/or record one or more of the following functions: agitation speed, aeration rate, headspace pressure, temperature, pressure regulation in exhaust air pipe and/or nutrient medium addition.

The recombinant bacteria and methods of the present invention may be particularly suitable for the production of target proteins which are typically difficult to produce using conventional protein expression processes involving bacteria. The target protein is preferably an exogenous protein. More preferably, it is hydrophobic and/or has a high propensity to form one or more beta-sheets. Thus, the target protein preferably can form at least one beta sheet, more preferably at least 2, 3, 4, 5, 6 or 7 beta sheets.

The present invention further provides the use of a recombinant bacterium selected from:
(a) the bacteria of the invention described above; and
(b) a bacterium selected from or derived (derivatives include mutants thereof) from E. *coli* strains K37, RR1 and W3110, such derivatives including strain W3110 which has been modified to contain a gene which encodes a polymerase and which is used the control of an inducible promoter (e.g. polymerase T7), in particular a bacterium having the identifying characteristics of strain MRE406.DE3 which has been deposited with the National Collection of Type Cultures (NCTC) of 61 Colindale Avenue, London, NW9 5HT, United Kingdom on 11 October 2005 by Mr M Raafat El-Gewely under the Budapest Treaty, or a mutant thereof, or a strain derived therefrom; in a method of large scale/industrial polypeptide or protein production.

The invention further provides a method of large scale/industrial recombinant polypeptide or protein production, the method comprising:
(i) providing a recombinant bacterium selected from:
   (a) the bacteria of the invention described above; and
   (b) a bacterium selected from or derived (derivatives include mutants thereof) from *E. coli* strains K37, RR1 and W3110, such derivatives including strain W3110 which has been modified to contain a gene which encodes a polymerase and which is used the control of an inducible promoter (e.g. polymerase T7), in particular a bacterium having the identifying characteristics of strain MRE406.DE3 which has been deposited with the National Collection of Type Cultures (NCTC) of 61 Colindale Avenue, London, NW9 5HT, United Kingdom on 11 October 2005 by Mr M Raafat El-Gewely under the Budapest Treaty, or a mutant thereof, or a strain derived therefrom; said bacterium containing a polynucleotide sequence coding for said recombinant polypeptide or protein;
(ii) culturing said bacterium under conditions suitable for expression of said recombinant polypeptide or protein; and optionally
(iii) recovering said recombinant polypeptide or protein.

Preferably, said recombinant polypeptide or protein is under the control of a promoter which can be recognised by said DNA polymerase.

In a further aspect the invention provides a bacterium having the identifying characteristics of strain MRE406.DE3 which has been deposited with the National Collection of Type Cultures (NCTC) of 61 Colindale Avenue, London, NW9 5HT, United Kingdom on 11 October 2005 by Mr M Raafat El-Gewely under the Budapest Treaty, or a mutant thereof, or a strain derived therefrom, and uses thereof in a method of recombinant protein production.

In a further aspect the invention provides a polypeptide or protein produced by the method of polypeptide or protein production of the invention.

In yet a further aspect the invention provides a method of generating a recombinant bacterium which comprises introducing nucleic acid which encodes a polymerase under the control of an inducible promoter into a bacterium selected from K37, RR1, W3110, a mutant of any thereof, and a strain derived from one of these strains with the proviso that where the bacterium is a W3110 strain it has the identifying characteristics of the strain deposited under number NCTC13370.

In yet a further aspect the invention provides a method of modifying a recombinant bacterium according to the present invention, which method comprises introducing nucleic acid coding for a target polypeptide or protein under the control of a promoter which can be recognised by an exogenous DNA polymerase into said bacterium.

The invention is described with reference to the Figures in which:
Fig. 1 is a graph showing the recombinant protein expression potential of different strains of E.coli using green fluorescent protein (GFP) as an indicator. The y axis in this graph shows the relative fluorescent intensity at 510nm per 10⁹ cells.
Fig. 2 is a diagrammatic representation of the reporter--3 plasmid that expresses green fluorescent protein constitutively. This plasmid (pGreen1) was isolated from bacterial cells expressing the green fluorescence protein without the IPTG induction.

The invention will now be further described in the following Examples.

### Example 1:

Introduction of the T7-polymerase gene into strains MRE403, MRE406 and MRE412.

Phage Lysates:
All phage lysates were purchased from Novagen.
λ-DE3
Helper Phage (λ-B10)
Selection Phage (λ-B482)
Tester Phage T7-4107

Procedures:
1- Single colony on LB plates from each strain was inoculated in 10ml culture of LB supplemented with 0.2% maltose and 10mM MgS04.
2- Shake at 37°C until the OD (at 600nm) is between 0.6 to 1 O.D. Cultures are cooled on ice.
3-Cells were centrifuged and resuspended in 2.5ml cold 10mM MgSO₄
4-The λ-DE3 Lysogenization Kit (Novagen) was used both for the lysogenization as well as for the testing of lysogenization.
5- A small volume containing 10⁸ pfu of the provided following phages stocks, lysogenization λ-DE3 (2.3x10¹⁰ pfu/ml) and Helper Phage (2.7x10¹⁰ pfu/ml) and Selection Phage (3.0x10¹⁰ pfu/ml) to 2µL of each strain in the 10mM MgSO₄ stocks.
6-The following Table summarizes volumes of the different phages added to cells stocks.

**Lysogenization Mix**

| No | Strain Used | λ-DE3 | Helper Phage λ-B10 | Selection Phage λ-B482 |
|---|---|---|---|---|
| 1 | MRE403 | 4.3 µL | 3.7 µL | 3.3 µL |
| 2 | MRE406 | 4.3 µL | 3.7 µL | 3.3 µL |
| 3 | MRE412 | 4.3 µL | 3.7 µL | 3.3 µL |

| | | | | |
|---|---|---|---|---|
| 6.1. Incubate cells with phage mixture at 37°C for 20 min. | | | | |

Add 100µL LB media to each tube
4.2. For plating to check surviving colonies (lysogenic for λ-DE3), each mix was added to one LB plate and cells were spread. Plates are Incubated at 37°C for overnight. Surviving cells should now be lysogenic for λ-DE3.
4.3. Several hundred cells / plate survived the selection. Only five colonies from each putative lysogenic strain were selected and grown at 37°C in 2ml LB supplemented with 0.2% maltose and 10mM MgS04 for overnight.
4.4. Cultures were chilled and centrifuged. Cells were resuspended in 750 µL cold 10mM MgS04.
4.5. In a small microfuge tubes 95µl diluted Tester Phage (10³/ml) was added to 95µl cell stock for each tested colony and incubated at 37°C for 10 min.
4.6. 3ml melted soft agar (0.6% agarose in LB; melted and kept at 46°C). Also add 50µl the induce IPTG (1M).
4.7 Incubate plate at 37°C overnight. The formed plaques indicate lysogenization in the presence of IPTG to facilitate the needed T7 polymerase.
4.8 Colonies that gave plaques using the "Tester Phage" (T7-4107) were selected.

5. These colonies were tested again for plaque formation as above, using the Tester Phage, but without the inducer IPTG. Colonies that gave the relatively smaller plaques, indicating less leaky expression of T7 polymerase in the absence of IPTG were selected. The final selected strains are named:
   MRE403.DE3 (NCTC13369)
   MRE406.DE3 (NCTC13370)
   MRE412.DE3 (NCTC13371)
   It was observed that in the absence of IPTG, strain MRE406.DE3 exhibited the smallest plaques. This might provide the least leaky strain to express target genes prior to adding the inducer IPTG.

8. Stocks of these new strains were frozen at -70°C.

### Example 2

### Evaluation of the protein expression ability of different bacterial strains.

A visual as well as a quantitative assay was used to measure the efficiency of production of functionally active protein by measuring the quantitative fluorescence of a reporter protein, using the plasmid pGreen1 (Figure 2). pGreen1 was isolated as a constitutive variant of pGreen-TIR (Miller and Lindow, 1997 Gene 191(2): 149-153). The reporter plasmid, pGreen1 uses the fluorescence emitting from green fluorescence protein as indication of the ability of the bacterial strain to produce functionally active protein.

The protocol used was as described by Miller and Lindow, supra. Briefly, pGreen-1 was introduced into several different bacterial strains. The transformed strains were cultured to allow expression of green fluorescent protein and the fluorescence intensity was measured.

The results are shown in Figure 1. It can be seen that the ability of the strains to produce functional protein varied significantly, with MRE403, MRE406 and MRE412 giving exceptional results.

## Claims

1. A recombinant bacterium selected from K37, RR1, W3110, a mutant of any thereof, and a strain derived from one of these strains, wherein said bacterium contains a gene which encodes an exogenous polymerase and which is under the control of an inducible promoter, with the proviso that where the bacterium is a W3110 strain it has the identifying characteristics of the strain deposited under number NCTC13370.

2. A recombinant bacterium according to claim 1, wherein said polymerase is T7 polymerase.

3. A recombinant bacterium according to claim 1 or 2, wherein said recombinant bacterium has the identifying characteristics of a strain selected from NCTC13369 and NCTC13371, a mutant of any thereof, and a strain derived from one of these strains.

4. Use of a recombinant bacterium according to any one of claims 1 to 3 in a method of recombinant polypeptide or protein production.

5. A method of recombinant polypeptide or protein production comprising the steps of
(a) introducing an expression vector into a recombinant bacterium according to any one of claims 1 to 3, the expression vector comprising a polynucleotide sequence coding for a target peptide or protein under the control of a promoter which can be recognised by said exogenous DNA polymerase;
(b) culturing the recombinant bacterium under conditions in which expression of said DNA polymerase is turned on; and
(c) recovering the recombinant protein.

6. A method of recombinant polypeptide or protein production comprising the steps of:
(a) providing a recombinant bacterium according to any one of claims 1 to 3 which additionally contains a polynucleotide sequence coding for a target polypeptide or protein under the control of a promoter which can be recognised by said exogenous DNA polymerase;
(b) culturing the recombinant bacterium under conditions in which expression of said DNA polymerase is turned on; and
(c) recovering the recombinant polypeptide or protein.

7. A method according to claim 5, wherein the expression vector contains a) a nucleotide sequence under the control of a promoter recognised by a specialised DNA polymerase, preferably T7 polymerase and at least one of the features selected from:
b) tRNA genes ArgU, ArgW and IleX to improve heterologous expression of eukaryotic proteins in a prokaryotic host cell; and/or
c) an origin of replication which is compatible with most other origins of replication; and/or
d) a selectable marker

8. A method according to claim 7, wherein said vector has two or more of features (b) to (d).

9. A method according to claim 8, wherein said vector has all of features (b) to (d).

10. A method according to claim 7, wherein the selectable marker is an antibiotic resistance gene.

11. A method of large scale/industrial recombinant polypeptide or protein production, the method comprising:
(i) providing a recombinant bacterium selected from K37, RR1, W3110, a mutant of any thereof, and a strain derived from one of these strains, wherein said bacterium contains a gene which encodes an exogenous polymerase and which is under the control of an inducible promoter
(ii) culturing said bacterium under conditions suitable for expression of said recombinant polypeptide or protein; and optionally
(iii) recovering said recombinant polypeptide or protein.

12. A method according to claim 11, wherein said method is carried out in a total culture volume of more than 5, 10, 50, 100, 200, 300, 400, 500, 600, 700, 800, 900, 1000, 1500, 2000, 2500 or 3000 L.

13. A method according to any one of claims 5-12, wherein the target polypeptide or protein is hydrophobic.

14. A method according to any one of claims 5-13, wherein the target polypeptide or protein contains at least one beta-sheet.

15. A polypeptide or protein produced by the method of any one of claims 5 to 14.

16. A method of generating a recombinant bacterium which comprises introducing nucleic acid which encodes a polymerase under the control of an inducible promoter into a bacterium selected from K37, RR1, W3110, a mutant of any thereof, and a strain derived from one of these strains with the proviso that where the bacterium is a W3110 strain it has the identifying characteristics of the strain deposited under number NCTC13370.

17. A method of modifying a recombinant bacterium as defined in any one of claims 1-3, which method comprises introducing nucleic acid coding for a target polypeptide or protein under the control of a promoter which can be recognised by an exogenous DNA polymerase into said bacterium.
